# EUROPEAN PATENT SPECIFICATION

(11) **EP 4 096 523 B1**
(45) Date of publication and mention of the grant of the patent: **24.01.2024**
(21) Application number: 21711344.8
(22) Date of filing: 08.03.2021
(51) Int. Cl.: A61B 5/15, A61B 5/153

(54) **SYRINGE APPARATUS AND SYRINGE ADAPTER DEVICE**
SPRITZENVORRICHTUNG UND SPRITZENADAPTERVORRICHTUNG
APPAREIL DE SERINGUE ET DISPOSITIF D'ADAPTATEUR DE SERINGUE

(30) Priority: 10.03.2020 GB 202003445
(43) Date of publication of application: 07.12.2022
(73) Proprietor: Medica Instrumentae Limited, Cheltenham GL50 3PG (GB)
(72) Inventor: BILKSTYS-RICHARDSON, Jokubas Gintaras, Cheltenham GL50 3PG (GB)
(74) Representative: Doherty, William
(86) International application number: PCT/GB2021/050569
(87) International publication number: WO 2021/181073

(56) References cited:
- WO-A1-03/068073
- WO-A1-2018/152335
- WO-A2-2012/020957
- US-B1- 6 368 308

## Description

The present invention relates to a syringe apparatus, particularly but not necessarily exclusively for improving the ease of use of syringes for the purpose of exsanguination. The invention further relates to a syringe adapter device, and syringe apparatus having such a syringe adapter device, as well as an integrated syringe apparatus capable of being used with one hand.

WO 03/068073 A1 describes an aspiration device comprising a plunger coupling element and a barrel coupling element for attaching a syringe, wherein the plunger coupling element comprises a thumb tip pressure portion at a rear end thereof and wherein the barrel coupling comprises a finger tip pressure portion at an end tip thereof.

When taking a blood sample from a patient, in a medical context in a process known as exsanguination or phlebotomy, a physician will usually engage a sterile needle with a corresponding syringe, find a suitable blood vessel, typically a vein in the arm, and then extract the blood sample.

A syringe is formed having a syringe body and a plunger, and retraction of the plunger with respect to the syringe body forms a negative pressure to draw up a blood sample from the vein. The physician must hold the syringe steady, so as to minimise damage or discomfort to the patient.

At present, the syringe must be operated with two hands; one hand on the syringe body, and the other on the plunger. It would be desirable, however, to have a means of stabilising the patient as well as the syringe, particularly for tremorous patients. If there is significant displacement between the patient and the needle, damage to the skin may occur, and indeed to the blood vessel itself. There is a significant risk of blood vessel collapse for smaller vessels. It is difficult, however, to modify the design of a syringe, since the mechanism is so simple. Modification of the syringe body could cause issues with leakage, as well as potentially hampering the sterility of the syringe.

The present invention seeks to provide a syringe adapter that can be used in conjunction with existing syringes to allow the syringe to be used in a one-handed fashion.

According to the invention, there is provided a syringe apparatus according to claim 1 comprising a syringe portion having a syringe or syringe holder adapted to receive a syringe therein, the syringe portion having a rear grip for seating in or against a user's hand and a front grip formed at or adjacent to a tip end of the syringe portion; and a movable member engagable with a plunger of the said syringe and a finger grip at or adjacent to a tip end thereof, the movable member being engaged with the syringe portion and configured to move relative to the rear grip when a user applies a force at the finger grip; wherein the front and rear grips are formed as lateral projections with respect to the movable member.

A syringe adapter device is a mechanism by which one-handed control of a syringe can be achieved without needing to modify the syringe itself. This allows a single device to be utilised with pre-existing syringes without a redesign of the syringe function. The rear grip provides a surface against which the one-handed device can be stabilised, whilst the movable member provides the means of retracting the plunger of the syringe in a comfortable manner without necessarily required the user's non-dominant hand. This allows the non-dominant hand to be used to stabilise the patient, or assist with blood vessel targeting, so that less damage is caused to the patient during the retrieval of a blood sample. The invention could also be extended to include a syringe having an integrated movable member, obviating the need to provide a separate syringe adapter device. The provision of a front grip dramatically improves the stability of the apparatus, since the user can use their thumb and/or middle finger to reinforce the needle end of the apparatus. This results in a significant reduction in velocity and displacement measurable at the syringe outlet, reducing damage or discomfort to the patient. Preferably, the movable member may be slidably engagable with the syringe portion.

A sliding mechanism for retracting the plunger has been found to be the most ergonomic and comfortable for use with a syringe or adapter device, since the direction of sliding is the natural axis along which the index finger operating the finger grip will move.

Optionally, the movable member may be at least in part receivable in a longitudinal channel of the syringe portion.

A longitudinal channel will encourage the movement direction of the movable member along the correct axis of the syringe or adapter, ensuring the movable member moves along the axis of the index finger as it slides.

The rear grip may preferably comprise at least one lateral flange palm grip.

A lateral extension would be the most comfortable means of providing buttressing of the palm by the syringe adapter device, since this will put the index finger in a suitable position in line with the axial direction of the plunger.

Preferably, the rear grip may comprise a pair of opposed said lateral palm grip portions. Having flange portions on both sides of the device will allow ambidextrous operation, and as such, different handed devices do not need to be created for left- and right-handed users. Optionally, the or each lateral flange portion may have a contoured profile.

The contoured profile of the rear grip improves the comfort when resting in the user's palm, which is important as the user will be urging the device against the palm when taking a blood sample.

The front grip may preferably have a lateral extent which is less than or equal to a lateral extent of the rear grip.

The front grips are primarily for stabilisation purposes, rather than providing significant anchoring against the motion of the plunger, and therefore do not need to be as large as the rear grips.

Where a syringe adapter device is provided, the movable member may be engaged with the syringe holder opposite to a syringe-receiving aperture of the syringe holder.

Installation of the syringe from below allows for the symmetric design of the syringe adapter device to be achieved. This creates a stable centre of gravity, improving stability, in addition to providing the ambidextrous design outlined above.

In an alternative embodiment, the movable member may be engaged with the syringe holder adjacent to a syringe-receiving aperture of the syringe holder.

A side entry for the syringe into the syringe holder may allow for a more compact product to be created, which may then be more cost-effective to manufacture.

Preferably, the movable member may include a plunger-connector for receivably coupling to the plunger.

Optionally, the plunger-connector may comprise a stop abuttably engagable with the syringe holder to limit travel of the engaged plunger. Additionally, or alternatively, the syringe portion may comprise a stop at or adjacent to a tip end thereof.

Stops for the movable member advantageously prevent overtravel thereof. This ensures that the syringe adapter device does not fall apart during use.

In one embodiment the syringe portion may be a syringe, and the movable member may be integrally formed with a syringe body of the syringe. Additionally, or alternatively, the movable member may be integrally formed with the plunger.

In an alternative embodiment, the syringe apparatus may be provided in the form of a syringe adapter device engagable with a syringe.

According to the invention, there is provided a syringe apparatu according to claim 15 comprising: a syringe having a syringe body and a plunger receivable in the syringe body; and a syringe adapter device; wherein engaging the syringe with the syringe adapter device allows a user to operate the syringe apparatus with one hand.

A one-handed operation of a syringe has the benefit of freeing the user's non-dominant hand for other tasks, such as patient stabilisation, and therefore allows for a less intrusive exsanguination procedure to be performed on the patient. This is particularly important for patients with tremors.

The invention will now be more particularly described, by way of example only, with reference to the accompanying drawings, in which:
Figure 1 shows an isometric representation of a syringe apparatus in accordance with the invention, comprising a syringe inserted into a syringe adapter;
Figure 2 shows a plan view from below of the syringe apparatus of Figure 1;
Figure 3 shows a plan view from above of the syringe apparatus of Figure 1;
Figure 4 shows a side view of the syringe apparatus of Figure 1;
Figure 5 shows an exploded view of the syringe and syringe adapter of Figure 1;
Figure 6 shows an isometric representation of the syringe apparatus of Figure 1, with the plunger of the syringe in a retracted condition;
Figure 7 shows a side perspective representation of the syringe apparatus of Figure 1 in an in use condition;
Figure 8 shows a perspective representation of an example of a syringe apparatus which is not in accordance with the invention, comprising a syringe inserted into a syringe adapter;
Figure 9 shows a side perspective representation of the syringe apparatus of Figure 8, in a retracted condition;
Figure 10A shows a perspective representation of an example of a syringe apparatus which is not in accordance with the invention, comprising a syringe inserted into a syringe adapter;
Figure 10B shows a perspective representation of the syringe apparatus of Figure 10A, in a retracted condition;
Figure 11A shows an isometric representation of an embodiment of a movable member of a syringe adapter;
Figure 11B shows an isometric representation of an embodiment of a movable member of a syringe adapter;
Figure 11C shows an isometric representation of an embodiment of a movable member of a syringe adapter;
Figure 11D shows an isometric representation of an embodiment of a movable member of a syringe adapter;
Figure 11E shows an isometric representation of an embodiment of a movable member of a syringe adapter; and
Figure 11F shows an isometric representation of an embodiment of a movable member of a syringe adapter.

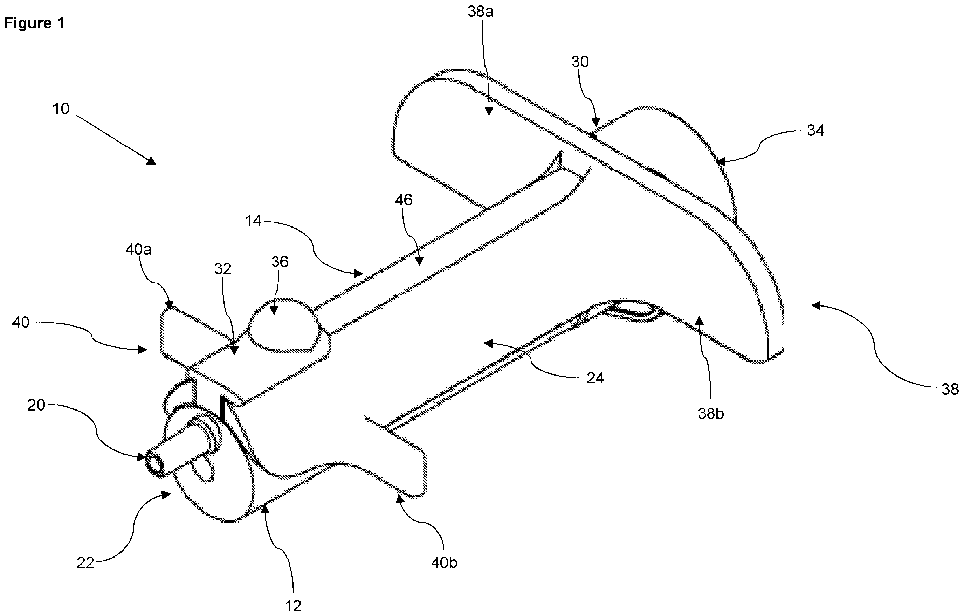

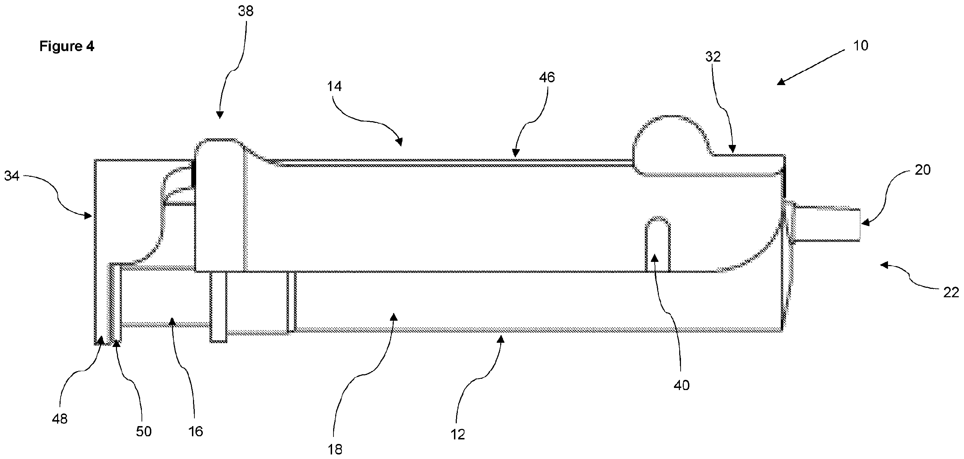

Referring to Figures 1 to 4, there is indicated a syringe apparatus, referenced globally at 10, and which is suited for taking blood samples from a patient using only one hand. The syringe apparatus 10 comprises two releasably engagable parts: a syringe 12, such as the plastic Luer-type syringes well-known in the art; and a syringe adapter device 14 which allows the syringe 12 to be held with one hand whilst also allowing the retraction of the plunger 16 of the syringe 12 with the same hand.

The syringe adapter device 14 is dimensioned to receive a syringe 12 of standard dimensions. Commonly used plastic syringes have volumes of 1ml, 2.5ml, 5ml, 10ml, and 25ml, with the 10ml volume being most commonly used in medical practice for taking blood samples.

It will be appreciated that whilst the indicative embodiments of the invention are shown as releasably engagable syringes and adapter devices, an integrally formed syringe apparatus could be provided in which the adapter device was inbuilt to the syringe.

The syringe 12 has a syringe body 18 within which the plunger 16 is receivable, and actuation of the plunger 16 with respect to the syringe body 18 changes the internal volume of the syringe 12 to either expel the contents of the syringe 12 through the syringe outlet 20 at a tip end 22 thereof, or alternatively draw matter into the syringe body 18 on retraction of the plunger 16. This is the context in which the syringe apparatus 10 might be used for taking blood samples.

The syringe adapter device 14 comprises a syringe holder 24 which has a syringe receiver 26 on one side thereof within which the syringe 12 is insertable. For the syringe adapter device 14, it will be appreciated that the syringe holder forms a syringe portion of the apparatus 10, that is, the area in which the syringe 12 can be located. For the embodiment of the invention described in more detail below, the syringe portion would be the syringe body 18 of the syringe.

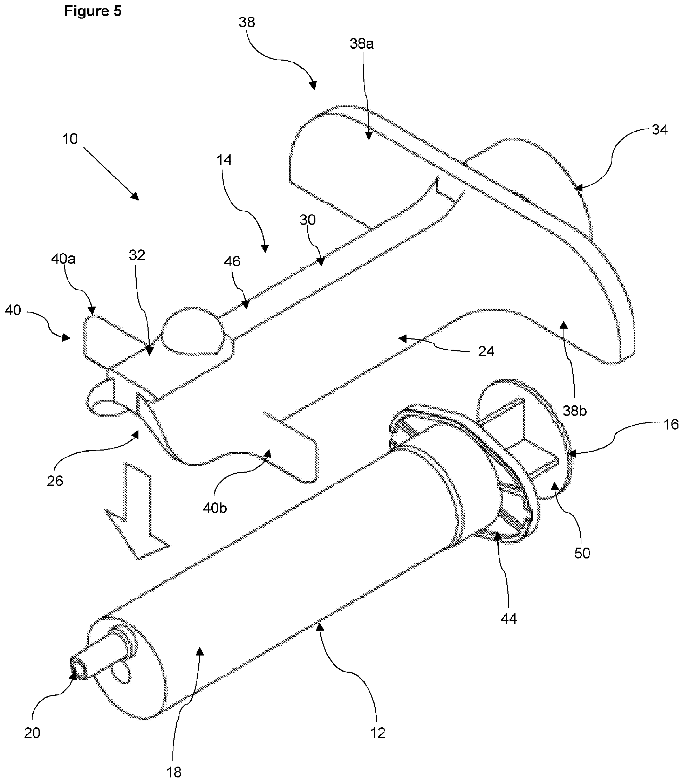

The engagement between the syringe 12 and the syringe adapter device 14 is indicated in Figure 5. The syringe holder 24 includes an elongate channel 28 extending along the length thereof, which is adapted to receive a movable member 30 therein. The movable member 30 includes a finger grip 32 at the tip end 22 of the syringe holder 24, and a plunger-connector 34 engagable with the plunger 16 of the syringe 12. The finger grip 32 is here formed as a slider tab having a nub 36 thereon to improve grip.

The syringe holder 24 includes a rear grip 38 which is receivable within the user's hand in use and which the user's palm abuts. Here, the rear grip 38 is provided as a pair of lateral palm grip portions 38a, 38b which oppose one another, having a contoured or curved upper edge for comfort. A single rear grip element may be provided, but the opposed flange portions ensure that the syringe adapter device 14 can be used with either hand with ease.

Lateral here refers to the fact that that, not only do the palm grip portions 38a, 38b extend in a direction which is perpendicular to the axis of motion of the plunger 16, but also that the palm grip portions 38a, 38b extend in a direction perpendicular to the side on which the finger grip 32 of the movable member 30 is positioned.

At or adjacent to the tip end 22 of the syringe holder 24 is a front grip 40 which is designed for engagement with a user's finger or thumb, providing forward stabilisation close to the needle point of the syringe 12. The front grip 40 may also be provided as one or more lateral projections, forming right- and/or left-hand front grips 40a, 40b, which are preferably angularly oriented to the lateral palm grip portions 38a, 38b of the rear grip 38. For comfort, the front grip 40 may have a width that is less than or equal to that of the rear grip 38. This orientation of the respective flange portions may simplify the manufacture of the syringe holder 24, for example, if created by injection moulding.

Again, lateral here refers to the fact that that, not only do the front grip portions 40a, 40b extend in a direction which is perpendicular to the axis of motion of the plunger 16, but also that the front grip portions 40a, 40b extend in a direction perpendicular to the side on which the finger grip 32 of the movable member 30 is positioned.

This achieves the arrangement of the front and rear grip portions 40a, 40b, 38a, 38b which permits the pencil grip. The front and rear grip portions 40a, 40b, 38a, 38b thus all intersect a nominal horizontal in-use plane of the syringe apparatus 10, which then by extension positions the finger grip 32 of the movable member 30 on a top surface of the syringe apparatus 10.

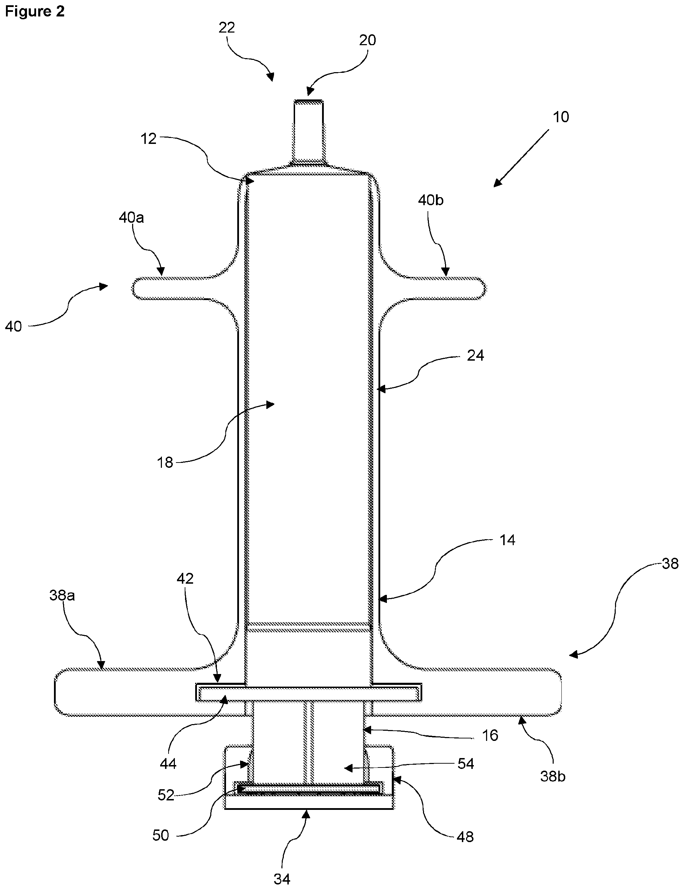

Figure 2 shows one method of ensuring engagement between the syringe 12 and the syringe adapter device 14. The syringe receiver 26 may be dimensioned to be tight fit to the diameter of the syringe 12, but may also comprise a flange receiver 42 into which a barrel flange 44 of the syringe 12 can be inserted. The flange receiver 42 may be housed in the rear grip 38 for structural stability.

The movable member 30 is an elongate member, having an elongate spine 46 which is dimensioned to fill or substantially fill the elongate channel 28 of the syringe holder 24 in a compact condition, that is, where the plunger 16 is fully received within the syringe body 18. In the embodiment shown, the plunger-connector 34 is formed as a cap 48 which is receivable about the plunger top 50 of the syringe 12, and a complementarily-shaped recess 52 may be provided in the cap 48 which accommodates both the neck 54 and plunger top 50. The cap 48 itself is preferably designed to envelop the perimeter of the plunger top 50, thereby holding it securely in place. This provides lateral security, so that the plunger 16 does not dislocate from the plunger-connector 34 in use, but also axial securing is or can be provided, to ensure that there is no lag between the movement of the movable member 30 and plunger 16.

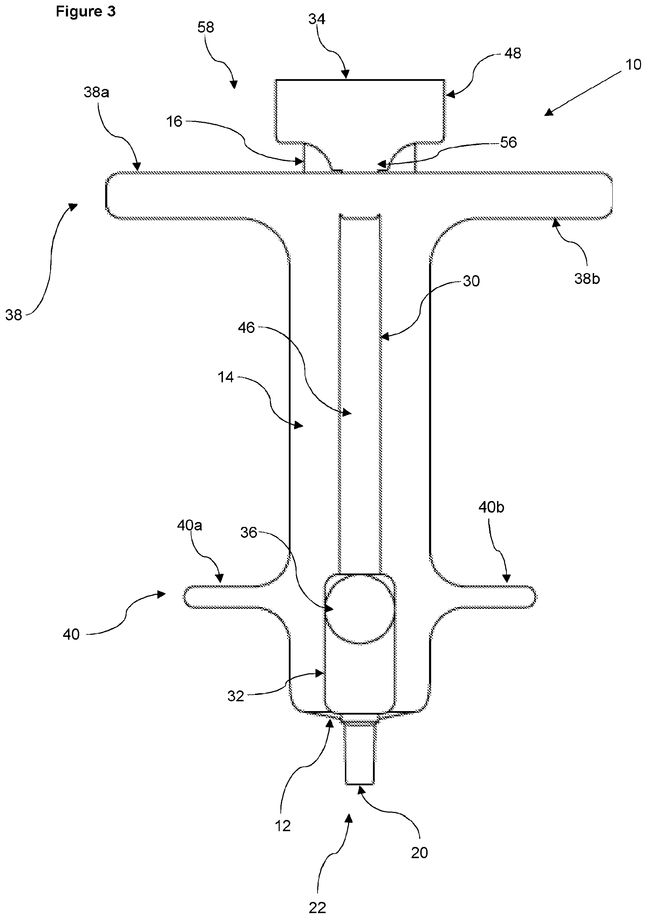

As is best seen in Figure 3, the interface between the plunger-connector 34 and the elongate spine 46 may form a stop 56 which is abuttably engagable with a plunger end 58 of the syringe holder 24 to prevent overextension of the plunger 16 into the syringe body 18. This may also advantageously result in the finger grip 32 meeting the tip end 22 of the syringe holder 24 flushly in the contracted condition.

The finger grip 32 itself may be dimensioned to overhang the elongate channel 28 on at least one, and preferably both, lateral directions thereof. This may improve the comfort of the use of the finger grip 32, since the user cannot get any part of their finger stuck in the elongate channel 28.

To engage the syringe 12 with the syringe adapter device 14, the syringe 12 needs to be placed into the syringe receiver 26, with the plunger top 50 being engaged with the plunger-connector 34. Stability of the syringe apparatus 10 may be improved if the syringe outlet 20 is positioned so as to be proximal to the syringe holder 24, as can be seen in Figure 5.

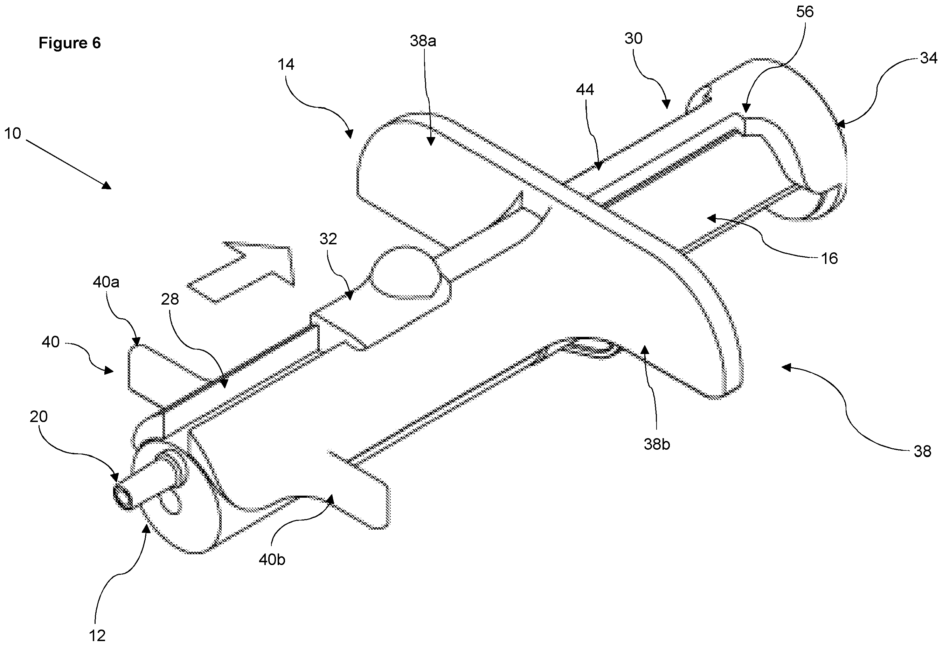

Once engaged, the finger grip 32 can be used to withdraw the plunger 16, as can be seen indicated by the motion arrow of Figure 6. Since the finger grip 32 and plunger-connector 34 are interconnected by the elongate spine 46, actuation of the finger grip 32 will urge the plunger-connector 34 simultaneously. This allows the drawing up of a sample, such as a blood sample during an exsanguination procedure, into the syringe body 18 to be effected simple using a one-handed motion of the user.

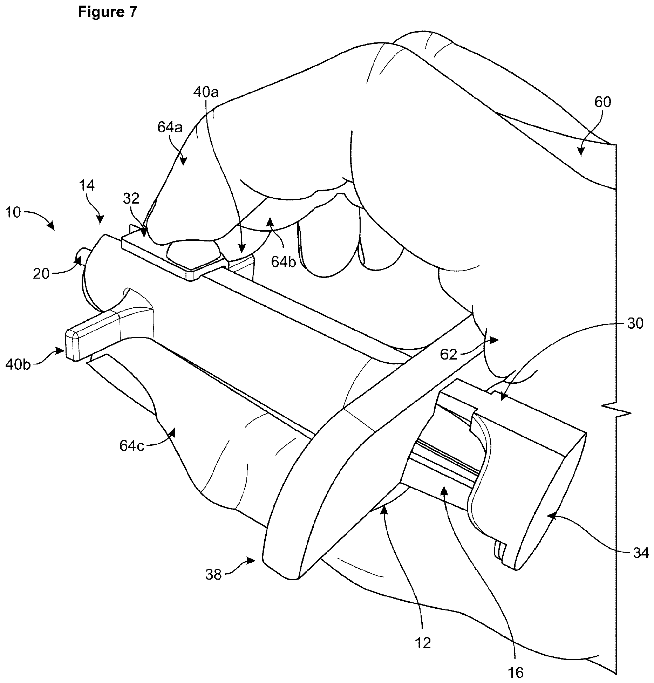

The user grip can be seen in Figure 7. The user here holds the syringe apparatus 10 in their right hand 60. The right-hand lateral flange portion 38 of the rear grip 38 is received in the user's hand 60 so as to abut the palm 62. The dimensions of the syringe holder 24 are such that the user's finger 64a can comfortably reach the distal finger grip 32 when the rear grip 38 is abuttably engaged with the user's palm 62, as the finger grip 32 is on an upper surface of the syringe apparatus 10 when the syringe apparatus 10 is held in this manner.

The shape of the syringe adapter device 14 is such that the index finger 64a will sit naturally on the finger grip 64a. The middle finger 64b can then be used as a stabiliser, either pressing on or hooking around the right-hand lateral front grip 40a, with the thumb 64c buttressing the left-hand lateral front grip 40b, as well as the syringe body 18 from below. It will be apparent to the skilled reader that the grip configuration will be reversed for the left-handed grip.

This creates a pencil or pincer grip. To take blood, the user holds the syringe apparatus 10 in one hand, and can then retract the index finger 64a so that the finger grip 32 slides the plunger 16 into the retracted condition.

Stabilisation of the front grip 40 using the thumb 64c and middle finger 64b will ensure minimal disruption of the needlepoint position when a needle is attached to the syringe, leading to a significant reduction in damage to the patient, particularly to their blood vessels such as veins, during exsanguination.

Extensive testing as to the stability improvements provided by the syringe adapter device 18 have been conducted.

### Comparative Example

For the comparative study, a 10ml syringe (BD (RTM) Biosciences, 1030 Eskdale Road, Winnersh Triangle, Wokingham, Berkshire, RG41 5TS, United Kingdom) was utilised for a test exsanguination process, using a 21 gauge needle (BD (RTM Biosciences) on a standard phlebotomy training arm (Muttiy Inc., (bangongchangsuo) 9002shi, Shangtangsongzaiyuan, 2qu, 51dong, Minzhijiedao, Baoanqu, Shenzhen, Guangdong, 518131, People's Republic of China), filled with cranberry juice to simulate blood.

Stability was measured with a Landtek VM6370 High Vibration Meter (Guangzhou Landtek Instrument Co., Ltd, Block C.Kengkou Electronic Base, No.9 Huaxi Road, Fang Village. Guangzhou, Guangdong 510380, People's republic of China) which measured acceleration, velocity, and displacement of the syringe outlet 20. Connection between the vibration meter and sensor was made with a 15cm cable provided by the manufacturer. A bespoke adapter for the vibration sensor was 3D printed and attached to the end of the syringe 12 and used for the duration of the experiment which kept the sensor in a fixed and stationary position while the syringe 12 was in use.

The aim of the experiment was to demonstrate that the use of the syringe adapter device 14 improved stability while taking blood, as well as having the syringe 12 in an easier manual configuration than is traditionally used, that is, with a one-handed configuration rather than a two-handed one. With the vibration meter connected to the syringe 12, the user would insert the needle into e vein site on the phlebotomy arm, after which the vibration meter was set to 'Hold'. This enabled the highest reading for each monitored variable to be read whilst measurements continued to be taken and recorded.

After flashback of the simulation blood was seen, the plunger 16 of the syringe 12 was pulled back. Throughout the duration of the drawing of the plunger 16, the highest values for acceleration, velocity and displacement were monitored and recorded. Approximately 5ml of fluid was drawn into the syringe 12 simulating the typical amount used in a clinical situation for full blood count (FBC) and urea and electrolyte (U&E) testing.

Thirteen trials were conducted for each of the three syringe configurations used: 1) a syringe 12 with no adapter, using two hands; 2) a modified syringe apparatus as described above, in which the front grip 40 was omitted; and 3) a syringe apparatus as described above, inclusive of the front grip 40.

**Table 1**

| **Control** | **Velocity (mm/s)** | **Acceleration (mm/s²)** | **Displacement (mm)** |
|---|---|---|---|
| | 3.33 | 0.47 | 0.272 |
| | 2.5 | 1 | 0.37 |
| | 3.23 | 0.1 | 0.177 |
| | 3.29 | 0.1 | 0.179 |
| | 3.01 | 1.4 | 0.283 |
| | 3.51 | 1.3 | 0.204 |
| | 4.66 | 0.4 | 0.436 |
| | 3.91 | 1.3 | 0.197 |
| | 4.35 | 0 | 0.072 |
| | 3.11 | 6.9 | 0.139 |
| | 2.76 | 1.7 | 0.179 |
| | 3.07 | 0.5 | 0.148 |
| | 5.23 | 5.3 | 0.57 |
| | 3.21 | 8.8 | 0.297 |
| **Average** | 3.782 | 2.251 | 0.271 |
| **Standard Deviation** | 0.764 | 2.799 | 0.133 |

**Table 2**

| **Syringe Apparatus 1** | **Velocity (mm/s)** | **Acceleration (mm/s²)** | **Displacement (mm)** |
|---|---|---|---|
| | 3.99 | 0.1 | 0.079 |
| | 3.89 | 0.1 | 0.098 |
| | 2.11 | 1.1 | 0.078 |
| | 2.69 | 0.1 | 0.162 |
| | 2.2 | 0.1 | 0.184 |
| | 2.95 | 4.8 | 0.28 |
| | 2.25 | 0.4 | 0.136 |
| | 1.81 | 0 | 0.133 |
| | 2.89 | 4 | 0.546 |
| | 2.61 | 1.2 | 0.338 |
| | 2.77 | 0.3 | 0.261 |
| | 2.46 | 0.2 | 0.305 |
| | 2.25 | 0.4 | 0.318 |
| | 2.98 | 0.3 | 0.131 |
| **Average** | 2.912 | 1.008 | 0.235 |
| **Standard Deviation** | 0.628 | 1.519 | 0.131 |

**Table 3**

| **Syringe Apparatus 2** | **Velocity (mm/s)** | **Acceleration (mm/s²)** | **Displacement (mm)** |
|---|---|---|---|
| | 1.59 | 0.2 | 0.169 |
| | 3.15 | 1.3 | 0.133 |
| | 1.57 | 0.2 | 0.115 |
| | 1.43 | 0.3 | 0.186 |
| | 1.33 | 0 | 0.037 |
| | 1.55 | 2.3 | 0.185 |
| | 2.59 | 2.3 | 0.103 |
| | 2.03 | 0.6 | 0.133 |
| | 1.19 | 0.8 | 0.117 |
| | 2.13 | 0.4 | 0.131 |
| | 1.13 | 0 | 0.123 |
| | 2.23 | 1.7 | 0.161 |
| | 1.75 | 0.6 | 0.111 |
| | 1.66 | 0.3 | 0.133 |
| **Average** | 1.948 | 0.846 | 0.141 |
| **Standard Deviation** | 0.565 | 0.799 | 0.038 |

### Results

Subjective results from testing with the syringe apparatuses 1 and 2 (respectively that without the front grip, and that described above and shown in Figures 1 to 7) have been very positive. Most users remark on the ease of use with one hand and the simplicity of the finger motion to enable drawing of the plunger 16 back compared to normal two-handed syringe usage. Additionally, the one-handed configuration enables an enhanced grip on the syringe 12 that is sometimes lost with a two-handed configuration when wetness or moisture is present depending on the clinical situation.

Most users also commented on the added stability that a one-handed configuration provides, not simply because the syringe apparatus 10 does not rely on two hands to activate the syringe adapter device 14 with the increased tension needed to operate it, but also that in certain situations where patients may have a resting tremor, having the non-dominant had at or near the venepuncture site also greatly facilitates stability.

Objective data obtained from an experienced user comparing the three configurations confirmed these subjective results, as outlined above. Velocity decreased for the first syringe apparatus when compared with the control syringe (3.78 mm/s versus 2.91 mm/s). This demonstrates the improvements achievable with the use of the 'pincer' grip that is afforded by the present invention.

Velocity further decreased for the second syringe apparatus 10 when compared with the first syringe apparatus (2.91 mm/s versus 1.94 mm/s) that allows for the use of a 'trigger' grip, in which the middle finger 64b hooks over the front grip 40 to hold back the syringe adapter device 14 and urge the rear grip 38 into the palm 62 of the user.

The velocity improvement for the enhanced syringe apparatus 10 showed a statistically significant deviation, such that the standard deviation bars for the control versus the second syringe apparatus 10 did not overlap.

Measurement of displacement followed a similar trend. The first syringe apparatus showed an improvement, that is, reduced displacement, over the control (0.234 mm versus 0.363 mm), and a further improvement between the second syringe apparatus 10 and the first syringe apparatus (0.141 mm versus 0.234 mm). Once again, there was no overlap between the standard deviation bars of the second syringe apparatus 10 and the control.

Statistical t-test analysis of the above study revealed that for velocity testing, all values were significantly different (t<0.05). For displacement testing, there was no statistically significant different in displacement between the first syringe apparatus and control (t=0.556), but a statistically significant difference was seen between the second syringe apparatus 10 and both the first syringe apparatus and control.

As such, not only does the one-handed syringe arrangement significantly improve stabilisation of the syringe apparatus 10 by reducing any velocity action at the needle point, but with the inclusion of the front grips, also demonstrably reduces displacement, limiting the risk of tearing of the patient's skin or blood vessels.

### Further examples

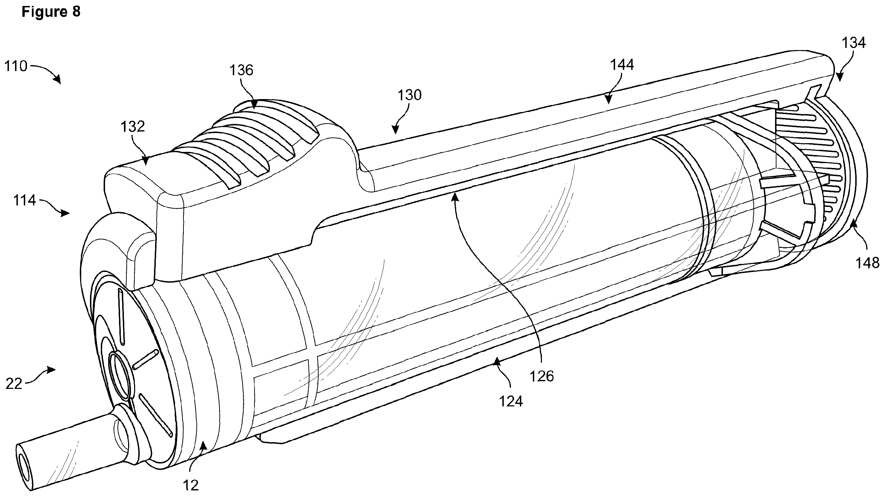

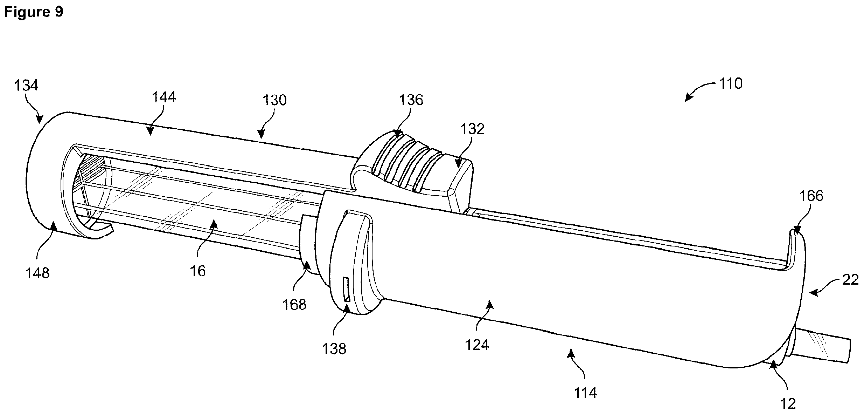

Other versions of the syringe apparatus could however, be considered. An example of the syringe apparatus is indicated globally at 110 in Figures 8 and 9. Identical or similar components to those of the first embodiment will be referenced using identical or similar reference numerals, and further detailed description will be omitted for brevity.

In this example, designed for the same syringe 12, the syringe adapter device 114 comprises a syringe receiver 126 which is positioned on the side of the syringe holder 124, thereby allowing a different means of entry for the syringe 12 therein.

The movable member 130 may therefore be exposed at or adjacent to the syringe receiver 126, and therefore the elongate spine 144 may not necessarily run in a longitudinal channel.

To ensure that the movable member 130 and syringe holder 124 do not dislocate from one another, the movable member 130 may be keyed to, or otherwise engaged with the syringe holder 124, to ensure that a smooth plunger 16 motion can be achieved.

Instead of the nub of the previous embodiment, the finger grip 132 may be formed so as to have a contoured and/or ergonomic portion 136 suited to receiving the tip of a user's index finger.

Since the syringe receiver 126 is positioned to one side of the syringe holder 124, the syringe apparatus 110 can no longer be used ambidextrously, and a rear grip 138 is only positioned on one side of the syringe holder 124. It will be apparent that a mirror-image syringe apparatus could be provided for left-hand operation.

The syringe holder 124 may have a forward stop 166, at or adjacent to the tip end 22 of the syringe 12, which may limit travel of the movable member 130 beyond the front of the syringe holder 124. A similar rear stop 168 may be provided at the opposite end of the syringe holder 124, which is engagable with the plunger-connector 134 of the movable member 130. This could be formed as an inner shoulder which slots into the cap 148 of the plunger-connector 134.

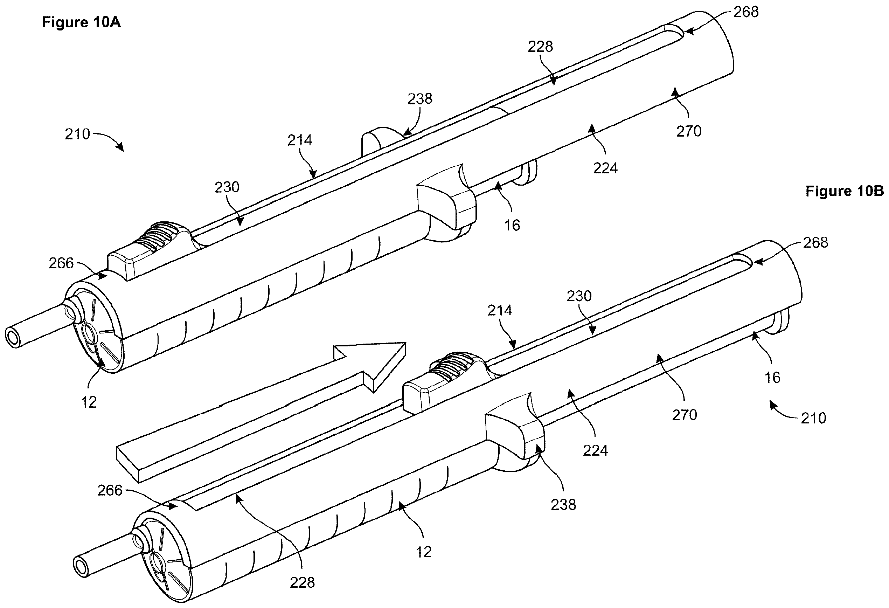

A third example of the syringe apparatus is indicated globally at 210 in Figures 10A and 10B. Identical or similar components to those of the first embodiment will be referenced using identical or similar reference numerals, and further detailed description will be omitted for brevity.

The syringe adapter device 214 has an elongated syringe holder 224 having an extended longitudinal channel 228 which fully encompasses the travel of the movable member 230. The longitudinal channel 228 therefore itself forms front and rear stops 266, 268 to prevent escape of the movable member 230 and allowing for free movement of the movable member 230 only within the longitudinal channel 228.

The syringe holder 224 therefore includes an extended rear portion 270 beyond the rear grip 238, which could be useful for resting against a user's arm, further stabilising the syringe apparatus 210. In this case, front grips may not be necessary.

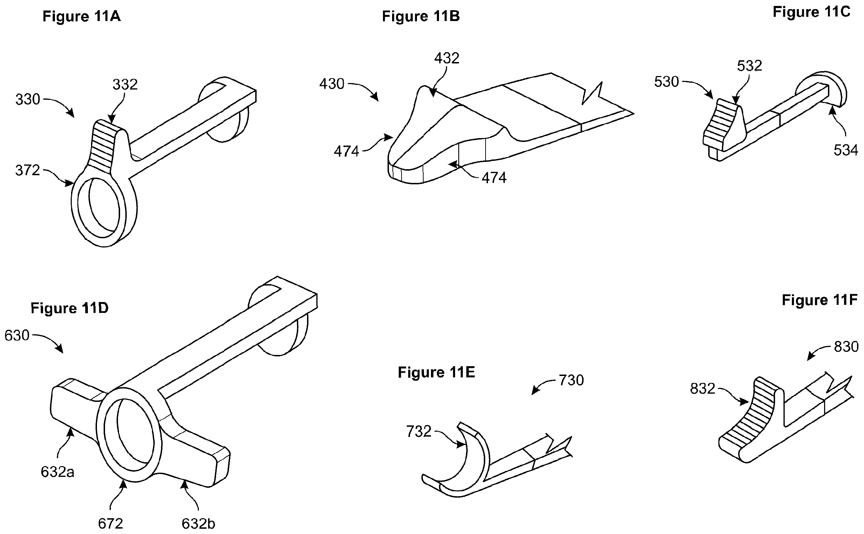

Various additional embodiments of the movable member are shown in Figures 11A to 11F. Identical or similar components to those of the first embodiment will be referenced using identical or similar reference numerals, and further detailed description will be omitted for brevity.

Figure 11A shows a movable member 330 having a contoured finger grip 332 which is at or adjacent to a front annulus 372 engagable with a front end of the plunger. This might assist with smooth and steady retraction of the plunger into the retracted condition.

Figure 11B shows a movable member 430 having a contoured finger grip 432, in which the lateral edges 474 of the finger grip 432 are shaped to receive a user's finger from the side.

Figure 11C shows a movable member 530 having a triangular finger grip 532. Instead of the full cap of the plunger-connector, a semi-circular plunger-connector 534 is provided, which seats around a part of the plunger top. This may assist with simple connection of the movable member 530 with the syringe.

Figure 11D shows a movable member 630 having lateral finger grips 632a, 632b. This may provide the user with a more powerful grip on the movable member 630, since a V-shaped finger grip can be assumed, with the index and middle fingers both engaging the lateral finger grips 632a, 632b respectively. The front annulus 672 is also shown here, but this embodiment could readily be used with the standard configuration of movable member 630.

Figure 11E shows a movable member 730 having a finger hook 732 in lieu of a nub or similar non-engaging grip. The user can insert their finger into the finger hook 732 to apply the actuating force to the movable member 730.

Figure 11F shows a movable member 830 having a finger grip 832 which is half-way to becoming a finger hook, having an arcuate front surface around which the user may hook their finger.

As previously noted, whilst a syringe adapter device is disclosed, the present invention is not intended to exclude the possibility of an integrated syringe including the movable member as previously described. To this end, it may be possible to provide a syringe apparatus comprising a syringe having a syringe body, thereby forming the syringe portion as opposed to the syringe holder of the syringe adapter device, and a plunger receivable in the syringe body, and a rear grip for seating in or against a user's palm. The movable member then has a plunger-connector, which may be integrally formed with the plunger itself, for engaging with a plunger and a finger grip at or adjacent to a tip end of the movable member. The movable member is engaged with the syringe body and configured to move relative to the rear grip when a user applies a force at the finger grip.

The movable member can be integrally formed with, or journaled within, the syringe body so as to slide relative to the syringe body, instead of a separate syringe holder. However, in all other considerations, the method of operation of the movable member is identical to that of a syringe adapter device.

The advantage of an integrated syringe embodiment is that non-standard volumetric configurations can be considered. The syringe adapter device is suited for compatibility with existing syringes known in the art. However, there are various adaptations to the syringe itself that could be made to improve user comfort in a one-handed grip arrangement.

In particularly, the elongate nature of the syringes in the art are configured so that they can be readily operated with two hands. However, a shorter syringe with a wider barrel could now be considered, since a second hand is not required in or around the area of the syringe body. As such, a squat syringe with an integrally formed movable member engaged with the plunger could be provided.

It is therefore possible to provide a syringe or an adapter therefor which allows the plunger action to be performed with one hand. This can be achieved by providing a movable member engaged with the plunger, and which is positioned for operation by an index finger of a user holding the syringe in a pencil or pincer grip. This is completely different to the two-handed operation necessitated with syringes in the art.

## Claims

1. A syringe apparatus (10) comprising:
a syringe portion having a syringe (12) or holder (24) adapted to receive a syringe (12) therein, the syringe portion having a rear grip (38) for seating in or against a user's hand and a front grip (40) formed at or adjacent to a tip end (22) of the syringe portion; and
a movable member (30) engagable with a plunger (16) of the said syringe (12), the movable member (30) having a finger grip (32) at or adjacent to a tip end (22) thereof, the movable member (30) being engaged with the syringe portion and configured to move relative to the rear grip when a user applies a force at the finger grip (32);
wherein the front and rear grips (40, 38) are formed as lateral projections (40a, 40b; 38a, 38b) with respect to the movable member (30).

2. A syringe apparatus (10) as claimed in claim 1, wherein the movable member (30) is slidably engagable with the syringe portion.

3. A syringe apparatus (10) as claimed in claim 1 or claim 2, wherein the movable member (30) is at least in part receivable in a longitudinal channel (28) of the syringe portion.

4. A syringe apparatus (10) as claimed in any one of the preceding claims, wherein the rear grip (38) comprises at least one lateral flange palm grip (38a, 38b).

5. A syringe apparatus (10) as claimed in claim 4, wherein the rear grip (38) comprises a pair of opposed said lateral palm grip portions (38a, 38b).

6. A syringe apparatus (10) as claimed in claim 4 or claim 5, wherein the or each lateral flange portion (38a, 38b) has a contoured profile.

7. A syringe apparatus (10) as claimed in any one of the preceding claims, wherein the front grip (40) has a lateral extent which is less than or equal to a lateral extent of the rear grip (38).

8. A syringe apparatus (10) as claimed in any one of the preceding claims, wherein the syringe portion comprises a syringe holder (24), and the movable member (30) is engaged with the syringe holder (24) opposite to a syringe-receiving aperture of the syringe holder (24), or wherein the syringe portion comprises a syringe holder, and the movable member is engaged with the syringe holder adjacent to a syringe-receiving aperture of the syringe holder.

9. A syringe apparatus (10) as claimed in any one of the preceding claims, wherein the movable member (30) includes a plunger-connector (34) for receivably coupling to the plunger (16).

10. A syringe apparatus (10) as claimed in claim 9, wherein the plunger-connector (34) comprises a stop (56) abuttably engagable with the syringe holder (24) to limit travel of the engaged plunger (16).

11. A syringe apparatus as claimed in any one of the preceding claims, wherein the syringe portion comprises a stop at or adjacent to a tip end thereof.

12. A syringe apparatus as claimed in any one of the preceding claims wherein the syringe portion is a syringe, and the movable member is integrally formed with a syringe body of the syringe.

13. A syringe apparatus as claimed in any one of the preceding claims, wherein the movable member is integrally formed with the plunger.

14. A syringe apparatus (10) as claimed in any one of claims 1 to 12 in the form of a syringe adapter device (14) engagable with a syringe (12).

15. A syringe apparatus (10) comprising:
a syringe (12) having a syringe body (18) and a plunger (16) receivable in the syringe body (18); and
a syringe adapter device (14) as claimed in claim 14;
wherein engaging the syringe (12) with the syringe adapter device (14) allows a user to operate the syringe apparatus (10) with one hand.

## Patentansprüche

1. Spritzenvorrichtung (10), umfassend:
einen Spritzenteil mit einer Spritze (12) oder Halterung (24), die dazu eingerichtet ist, eine Spritze (12) darin aufzunehmen, wobei der Spritzenteil einen hinteren Griff (38) zum Lagern in oder gegen eine Hand eines Benutzers und einen vorderen Griff (40), der an oder benachbart zu einem Spitzenende (22) des Spritzenteils ausgebildet ist, aufweist, und
ein bewegliches Element (30), das mit einem Kolben (16) der besagten Spritze (12) in Eingriff gebracht werden kann, wobei das bewegliche Element (30) einen Fingergriff (32) an oder benachbart zu einem Spitzenende (22) davon aufweist, wobei das bewegliche Element (30) mit dem Spritzenteil im Eingriff steht und dazu konfiguriert ist, sich relativ zu dem hinteren Griff zu bewegen, wenn ein Benutzer eine Kraft an dem Fingergriff (32) ausübt;
wobei der vordere und der hintere Griff (40, 38) als seitliche Vorsprünge (40a, 40b; 38a, 38b) in Bezug auf das bewegliche Element (30) ausgebildet sind.

2. Spritzenvorrichtung (10) nach Anspruch 1, wobei das bewegliche Element (30) verschiebbar mit dem Spritzenteil in Eingriff gebracht werden kann.

3. Spritzenvorrichtung (10) nach Anspruch 1 oder Anspruch 2, wobei das bewegliche Element (30) zumindest zum Teil in einem Längskanal (28) des Spritzenteils aufgenommen werden kann.

4. Spritzenvorrichtung (10) nach einem der vorhergehenden Ansprüche, wobei der hintere Griff (38) mindestens einen seitlichen Flanschhandflächengriff (38a, 38b) umfasst.

5. Spritzenvorrichtung (10) nach Anspruch 4, wobei der hintere Griff (38) ein Paar von entgegengesetzten der seitlichen Handflächengriffteile (38a, 38b) umfasst.

6. Spritzenvorrichtung (10) nach Anspruch 4 oder Anspruch 5, wobei der oder jeder seitliche Flanschteil (38a, 38b) ein konturiertes Profil aufweist.

7. Spritzenvorrichtung (10) nach einem der vorhergehenden Ansprüche, wobei der vordere Griff (40) eine seitliche Erstreckung aufweist, die kleiner als oder gleich einer seitlichen Erstreckung des hinteren Griffs (38) ist.

8. Spritzenvorrichtung (10) nach einem der vorhergehenden Ansprüche, wobei der Spritzenteil eine Spritzenhalterung (24) umfasst und das bewegliche Element (30) mit der Spritzenhalterung (24) entgegengesetzt zu einer Spritzenaufnahmeöffnung der Spritzenhalterung (24) im Eingriff steht, oder wobei der Spritzenteil eine Spritzenhalterung umfasst und das bewegliche Element mit der Spritzenhalterung benachbart zu einer Spritzenaufnahmeöffnung der Spritzenhalterung im Eingriff steht.

9. Spritzenvorrichtung (10) nach einem der vorhergehenden Ansprüche, wobei das bewegliche Element (30) einen Kolbenverbinder (34) zum aufnehmbaren Koppeln an den Kolben (16) beinhaltet.

10. Spritzenvorrichtung (10) nach Anspruch 9, wobei der Kolbenverbinder (34) einen Anschlag (56) umfasst, der anliegend mit der Spritzenhalterung (24) in Eingriff gebracht werden kann, um eine Bewegung des im Eingriff stehenden Kolbens (16) einzuschränken.

11. Spritzenvorrichtung nach einem der vorhergehenden Ansprüche, wobei der Spritzenteil einen Anschlag an oder benachbart zu einem Spitzenende davon umfasst.

12. Spritzenvorrichtung nach einem der vorhergehenden Ansprüche, wobei der Spritzenteil eine Spritze ist und das bewegliche Element integral mit dem Spritzenkörper der Spritze ausgebildet ist.

13. Spritzenvorrichtung nach einem der vorhergehenden Ansprüche, wobei das bewegliche Element integral mit dem Kolben ausgebildet ist.

14. Spritzenvorrichtung (10) nach einem der Ansprüche 1 bis 12 in der Form einer Spritzenadaptervorrichtung (14), die mit einer Spritze (12) in Eingriff gebracht werden kann.

15. Spritzenvorrichtung (10), umfassend:
eine Spritze (12) mit einem Spritzenkörper (18) und einem Kolben (16), der in dem Spritzenkörper (18) aufgenommen werden kann, und
eine Spritzenadaptervorrichtung (14) nach Anspruch 14;
wobei ein Bringen der Spritze (12) in Eingriff mit der Spritzenadaptervorrichtung (14) einem Benutzer ermöglicht, die Spritzenvorrichtung (10) mit einer Hand zu bedienen.

## Revendications

1. Appareil de seringue (10) comprenant :
une partie seringue ayant une seringue (12) ou un support (24) adapté pour recevoir une seringue (12) en son sein, la partie seringue ayant une prise arrière (38) pour la mise en place dans ou contre la main d'un utilisateur et une prise avant (40) formée au niveau ou à proximité d'une extrémité de bout (22) de la partie seringue ; et
un élément mobile (30) pouvant venir en prise avec un piston (16) de ladite seringue (12), l'élément mobile (30) ayant une prise pour doigt (32) au niveau ou à proximité d'une extrémité de bout (22) de celui-ci, l'élément mobile (30) étant en prise avec la partie seringue et configuré pour se déplacer par rapport à la prise arrière lorsqu'un utilisateur applique une force au niveau de la prise pour doigt (32) ;
dans lequel les prises avant et arrière (40, 38) sont formées en tant que protubérances latérales (40a, 40b ; 38a, 38b) par rapport à l'élément mobile (30).

2. Appareil de seringue (10) selon la revendication 1, dans lequel l'élément mobile (30) peut venir en prise de manière coulissante avec la partie seringue.

3. Appareil de seringue (10) selon la revendication 1 ou la revendication 2, dans lequel l'élément mobile (30) peut au moins en partie être reçu dans un canal longitudinal (28) de la partie seringue.

4. Appareil de seringue (10) selon l'une quelconque des revendications précédentes, dans lequel la prise arrière (38) comprend au moins une prise palmaire latérale à rebord (38a, 38b).

5. Appareil de seringue (10) selon la revendication 4, dans lequel la prise arrière (38) comprend une paire desdites parties de prise palmaire latérale opposées (38a, 38b).

6. Appareil de seringue (10) selon la revendication 4 ou la revendication 5, dans lequel la ou chaque partie de rebord latéral (38a, 38b) a un profil profilé.

7. Appareil de seringue (10) selon l'une quelconque des revendications précédentes, dans lequel la prise avant (40) a une étendue latérale qui est inférieure ou égale à une étendue latérale de la prise arrière (38).

8. Appareil de seringue (10) selon l'une quelconque des revendications précédentes, dans lequel la partie seringue comprend un support de seringue (24), et l'élément mobile (30) est en prise avec le support de seringue (24) opposé à une ouverture de réception de seringue du support de seringue (24), ou dans lequel la partie seringue comprend un support de seringue, et l'élément mobile est en prise avec le support de seringue à proximité d'une ouverture de réception de seringue du support de seringue.

9. Appareil de seringue (10) selon l'une quelconque des revendications précédentes, dans lequel l'élément mobile (30) comprend un raccord de piston (34) pour accouplement par réception au piston (16).

10. Appareil de seringue (10) selon la revendication 9, dans lequel le raccord de piston (34) comprend un arrêt (56) pouvant venir en prise en butée avec le support de seringue (24) pour limiter un déplacement du piston en prise (16).

11. Appareil de seringue selon l'une quelconque des revendications précédentes, dans lequel la partie seringue comprend un arrêt au niveau ou à proximité d'une extrémité de bout de celle-ci.

12. Appareil de seringue selon l'une quelconque des revendications précédentes dans lequel la partie seringue est une seringue, et l'élément mobile est formé d'un seul tenant avec un corps de seringue de la seringue.

13. Appareil de seringue selon l'une quelconque des revendications précédentes, dans lequel l'élément mobile est formé d'un seul tenant avec le piston.

14. Appareil de seringue (10) selon l'une quelconque des revendications 1 à 12 sous la forme d'un dispositif adaptateur de seringue (14) pouvant venir en prise avec une seringue (12).

15. Appareil de seringue (10) comprenant :
une seringue (12) ayant un corps de seringue (18) et un piston (16) pouvant être reçu dans le corps de seringue (18) ; et
un dispositif adaptateur de seringue (14) selon la revendication 14 ;
dans lequel une mise en prise dans la seringue (12) avec le dispositif adaptateur de seringue (14) permet à un utilisateur de faire fonctionner l'appareil de seringue (10) à une main.
